# EUROPEAN PATENT SPECIFICATION

(11) **EP 4 522 585 B1**
(45) Date of publication and mention of the grant of the patent: **08.04.2026**
(21) Application number: 24758028.5
(22) Date of filing: 25.07.2024
(51) Int. Cl.: C07C 45/75, C07C 49/17, C07D 333/36

(54) **PROCESS FOR THE PREPARATION OF BETA-HYDROXYKETONES**
VERFAHREN ZUR HERSTELLUNG VON BETA-HYDROXYKETONEN
PROCÉDÉ DE PRÉPARATION DE BÊTA-HYDROXYCÉTONES

(30) Priority: 25.07.2023 CN 202310920422
(43) Date of publication of application: 19.03.2025
(73) Proprietor: Adama Agan Ltd., 7710001 Ashdod (IL)
(72) Inventor: WANG, Kai, Shunyi District Beijing 101300 (CN); CHEN, Jianguo, Minhang District Shanghai (CN); YACOVAN, Avihai, 7685919 Mazkeret Batya (IL); BAR-NAHUM, Itsik, 7691000 Kfar-Gibton (IL)
(74) Representative: ABG Intellectual Property Law, S.L.
(86) International application number: PCT/IL2024/050736
(87) International publication number: WO 2025/022398

(56) References cited:
- CN-A- 113 024 505
- CN-A- 118 146 187
- US-A1- 2020 010 395
- MORGAN G.T. ET AL: "Formaldehyde Condensations with Aliphatic Ketones. Part I", J. CHEM. SOC., 1932, pages 2667 - 2673, XP093214855, Retrieved from the Internet <URL:https://pubs.rsc.org/en/content/articlepdf/1932/jr/jr9320002667>

## Description

### FIELD OF THE INVENTION

The present invention relates to the field of synthesis of organic compounds, more specifically to a process for the preparation of beta-hydroxyketones that are useful in the preparation of dimethenamid.

### BACKGROUND PRIOR ART

The agrochemical industry is always in the search of more efficient processes for the preparation of its active ingredients (Als). The capability of providing economical and clean synthesis of the active ingredients is one of the key factors determining the commercialization of an active ingredient.

Dimethenamid is a herbicide belonging to the group of chloroacetamides, that inhibits lipid synthesis. It is included in group 15 of the WSSA classification. It is typically applied on the soil to control a variety of broad-leaved weeds and grasses. It is a chiral molecule having two isomeric forms commonly known as M and P stereoisomers, dimethenamid-P being more biologically active.

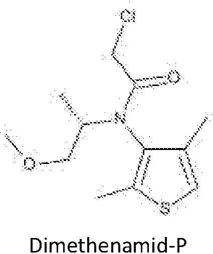

Synthetic schemes to prepare dimethenamid typically involve constructing the thiophene ring (typically a thiophen-3-one), followed by incorporating the 2-methoxy-2-propanamine (or 2-methoxyisopropylamine), also known as MOIPA, to finalize by coupling the 2-chloroaceto moiety. In the case of dimethenamid, the thiophen-3-one needed is 2,4-dimethyl-2,3-dihydrothiophen-3-one (also found in the literature as 2,4-dimethylthiophen-3-one). One possible route to this intermediate starts with 3-pentanone, which is then transformed into 1-hydroxy-2-methylpentan-3-one (a beta-hydroxyketone), which leads to 2,4-dimethyl-2,3-dihydrothiophen-3-one by halogenation and subsequent cyclization in the presence of a sulfide.

This process is disclosed in US 5,703,248, which in example 1 teaches how to prepare 1-hydroxy-2-methylpentan-3-one from 3-pentanone and paraformaldehyde in the presence of catalytic amounts of a base. According to US 5,703,248 formaldehyde is added as paraformaldehyde and no addition of water is reported and triethyl amine in catalytic amounts is used as base.

US 2020/0010395 discloses a process for the preparation of 1-hydroxy-2-methylpentan-3-one using the same aldol reaction but in the presence of water. US 2020/0010395 teaches the preparation of 1-hydroxy-2-methylpentan-3-one by aldol condensation of 3-pentanone and formaldehyde in the presence of a trialkylamine (e.g. triethyl amine) and water. According to US 2020/0010395 the reaction proceeds with better yields when higher amounts of trialkylamine are used.

In Morgan, G.T et al "Formaldehyde Condensations with Aliphatic Ketones. Part I", J. Chem. Soc., 1932, pages 2667-2673, the authors discuss the condensation of formaldehyde with diethylketone. The reaction proceeds in the presence of large amounts of alcohol to ensure a monophasic reaction mixture. The yield of the monocondensation product is low and obtains a large proportion of by-products from the double or triple condensation of formaldehyde.

CN 113 024 505 A discloses a preparation method of refined dimethenamid involving condensation of a thiophen-3-one ring with (2S)-1-hydroxypropyl-2-amine, followed by methylation and incorporation of chloroacetyl chloride.

There is therefore in the art a need to provide alternative procedures for obtaining dimethenamid and its intermediate beta-hydroxyketone. There is a need for processes that provide 1-hydroxy-2-methylpentan-3-one more efficiently, for example improving yields, reducing impurities, and/or milder conditions.

### DESCRIPTION OF FIGURES

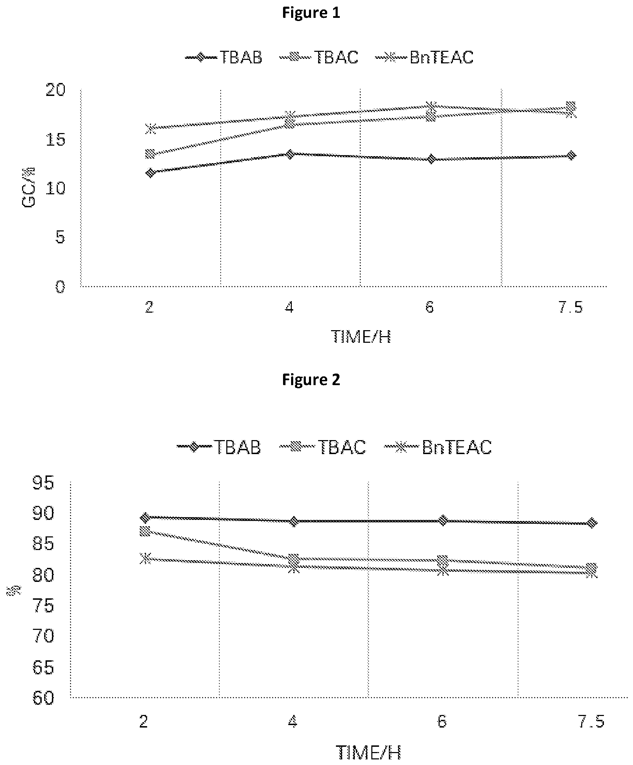

Figure 1: Graph of the starting 3-pentanone consumed in the reactions of Example 3.
Figure 2: Graph of the selectivity towards a compound of formula (I) in the reactions of Example 3. Selectivity at each point is calculated according to the following equation: X/(100-Y). X is the percentage of the desired product 1-hydroxy-2-methylpentan-3-one in the reaction mixture, according to the area under the peak in the IPC. Y is the percentage of remaining starting material with respect to the initial amount (in example 3 it is the remaining amount of 3-pentanone). For example, if the content of 1-hydroxy-2-methylpentan-3-one in the mixture is 12% and the remaining 3-pentanone is 87%, the selectivity is 12/(100-87), that is 92%.

### SUMMARY OF THE INVENTION

The process disclosed in US 5,703,248 has been found difficult to reproduce and unsuitable for large scale production. The inventors have now realized that the preparation of *beta-*hydroxyketones is improved by the presence of a phase transfer catalyst and water, and contrary to the teachings of US 2020/0010395, the use of catalytic amounts of bases result in good yields and selectivity, and small amounts of impurities. All attempts to reproduce the procedure of example 1 of US 5,703,248 failed to convert 3-pentanone into 1-hydroxy-2-methylpentan-3-one, as shown in the examples below.

Thus, a first aspect of the invention is a process for the preparation of a compound of formula (I) or a salt thereof, 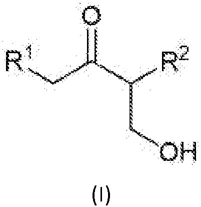
wherein each of R¹ and R² is selected from the group consisting of hydrogen, C₁-C₁₂
alkyl, C₂-C₁₂ alkenyl, C₂-C₁₂ alkynyl, C₆-C₁₅ aryl, C₂-C₁₅ heterocyclyl and C₇-C₁₅ arylalkyl; the process comprising contacting a base, a phase transfer catalyst, formaldehyde and water with a compound of formula (II) 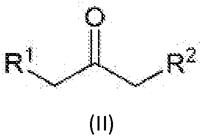
wherein R¹ and R² are as defined above.

The compound of formula (I) encompasses 1-hydroxy-2-methylpentan-3-one, necessary to prepare dimethenamid. It is therefore a further aspect of the invention a process for preparing dimethenamid comprising (i) contacting a base, a phase transfer catalyst, formaldehyde and water with 3-pentanone; and (ii) transforming the 1-hydroxy-2-methylpentan-3-one obtained in the previous step into dimethenamid.

The preparation of dimethenamid can be achieved in different ways. The compound of formula (I) (1-hydroxy-2-methylpentan-3-one) can be halogenated to produce 1,2,4-trichloro-2-methylpentan-3-one, which is then transformed into 2,4-dimethyl-2,3-dihydrothiophen-3-one in the presence of a disulfide. 2,4-dimethyl-2,3-dihydrothiophen-3-one can be reacted with 1-methoxy-2-propylamine to produce N-(1-methoxyprop-2-yl)-2,4-dimethylaminothiophene, which can then be reacted with the acyl chloride of 2-chloroacetate to yield dimethenamid. Examples of this procedure and other alternative schemes to dimethenamid are described in US 5,703,248, US 5,457,085, EP210320, EP296463, CN108299221 or CN113024505. Therefore, the process of the invention preferably comprises contacting a base, a phase transfer catalyst, formaldehyde and water with 3-pentanone.

The process of the invention provides good yields, less by-products, and easy to run, using inexpensive materials that are easy to separate from the final product.

### DETAILED DESCRIPTION OF THE INVENTION

### Definitions

In the present document the following terms are given the meaning below.

The compounds of the invention are also meant to include compounds which differ only in the presence of one or more isotopically enriched atoms. For example, compounds having the same structures as described herein but wherein a hydrogen atom has been replaced with deuterium or tritium, or a carbon atom has been replaced with a ¹³C- or ¹⁴C-enriched carbon, are within the scope of this invention.

The invention also provides salts of compounds of the compounds of formula (I), for example, base addition salts or metallic salts, which can be synthesized from the parent compound of formula (I) by conventional chemical methods. Examples of the base addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "C₁-C₁₂ alkyl" means a linear or branched saturated hydrocarbon chain radical having no multiple bonds and having from one to twelve carbon atoms, and which is attached to the rest of the molecule by a single bond. Suitable groups include, but are not limited to alkyl groups such as methyl, ethyl, propyl (e.g. n-propyl or iso-propyl), butyl (e.g. n-butyl, t-butyl, sec-butyl), pentyl (e.g. 1-methylpentyl, 3-methylpentyl, n-pentyl), hexyl, octyl, or dodecyl.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl), hexenyl (e.g. 1-hexenyl, 2-hexenyl, 3-hexenyl, 4-hexenyl, 5-hexenyl), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl), 2-propyl-2-butenyl, 4,6-Dimethyl-oct-6-enyl.

The term "C₂-C₁₂ alkynyl" means a linear or branched hydrocarbon chain radical having one or more carbon-carbon triple bonds therein and from two to twelve carbon atoms, and which is attached to the rest of the molecule by a single bond. The triple bond of an alkynyl group can be unconjugated or conjugated to another unsaturated group. Suitable alkynyl groups include, but are not limited to alkynyl groups such as ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl), hexynyl (e.g. 1-hexynyl, 2-hexynyl, 3-hexynyl, 4-hexynyl, 5-hexynyl), methylpropynyl, 3-methyl-1-butynyl, 4-methyl-2-heptynyl , and 4-ethyl-2-octynyl.

"C₆-C₁₅ Aryl" refers to a hydrocarbon moiety having six to fifteen carbon atoms and at least one aromatic hydrocarbon structure, such as phenyl, naphthyl or anthracyl.

"C₇-C₁₅ Arylalkyl" refers to an aryl group linked to the rest of the molecule by an alkyl group, such as benzyl and phenethyl.

"C₂-C₁₃ Heterocyclyl" refers to a stable 3- to 16- membered ring which consists of carbon atoms (between 2 and 15) and from one to five heteroatoms selected from the group consisting of nitrogen, oxygen, and sulphur, preferably a 4-to 8-membered ring with one or more heteroatoms, more preferably a 5- or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, or tetrahydrofuran.

The reaction does not necessarily require special equipment and any vessel typically used for chemical reactions should be appropriate. In case some pressure is expected to build up, autoclave vessels or other measures to deal with pressure can be used. The process can run in batch, a semi-batch or a continuous mode, preferably in batch mode.

The process of the invention is an example of the well-known base-catalyzed aldol reaction. Aldol reactions comprise the condensation between molecules containing aldehydes and ketones, at least one of them comprising a hydrogen atom in the *alpha* position relative to the carbonyl. As a result of the condensation, beta-hydroxyaldehydes and beta-hydroxyketones are formed. In the process of the invention beta-hydroxyketones are produced. Typically, the formation of the beta-hydroxyketones is accompanied by different impurities resulting from the multiple additions of formaldehyde to a single molecule of starting ketone or to other beta-hydroxyketones already formed. In this way, by products such as 1,5-dihydroxy-2,4-dimethylpentan-3-one or 1,5-dihydroxy-2-hydroxymethyl-2,4-dimethylpentan-3-one are formed. Also, aldol condensation faces the problem of by-products coming from the dehydration of the desired beta-hydroxyketone and of the by-products, such as, for example, 2-methyl-1-penten-3-one or 5-hydroxy-2,4-dimethyl-1-penten-3-one. This problem becomes especially acute at higher temperatures. These impurities reduce the yield, consume valuable starting materials and require additional, many times complex, work up and purification processes.

The inventors have found that the above problems are significantly reduced when the reaction proceeds in the presence of a phase transfer catalyst (PTC) and water. The inventors have tested different phase transfer catalysts, all of which have provided good results. The presence of a PTC reduces dehydration by-products as well as undesired compounds produced by the reaction of a compound of formula (I) with more than one molecule of formaldehyde. Even though US 2020/0010395 also uses water in the process, it is silent about the use of PTCs. In fact, it teaches against the use of biphasic systems because, according to US 2020/0010395, they increase the formation of by-products. Examples of PTCs are ammonium and phosphonium compounds, for example, those selected from the group consisting of tetraalkyl ammonium salts, trialkyl aralkylammonium salts or tetraalkyl-phosphonium salts such as tetrabutylammonium bromide (TBAB) or chloride (TBAC), tetramethylammonium chloride (TMAC), tetraethylammonium chloride (TEAC), methyl trioctylammonium bromide, methyl tributylammonium chloride, triethyl benzyl ammonium bromide or chloride (also known as benzyltriethylammonium bromide or chloride, respectively), and benzyl dodecyl dimethyl ammonium chloride (known as Zephirol^{®}). Typically, the phase transfer catalyst will be benzyltriethylammonium chloride (BnTEAC). Numerous brands exist, such as the Aliquat^{®} series. The phase transfer catalyst can be a single species such as methyl tributylammonium chloride (Aliquat^{®} 175) or a mixture such as Aliquat^{®} 336 (Stark's catalyst), which is quaternary ammonium salt that contains a mixture of octyl and decyl chains.

The phase transfer catalyst is added in small molar amounts relative to the formaldehyde. For example, it can be added in molar equivalent amounts of 0.001 to 1.0, with respect to the molar amount of formaldehyde, although it is typically added in molar amounts below those of formaldehyde, for example, in molar equivalent amounts of 0.002 to 0.5, or in molar equivalent amounts of 0.003 to 0.3, or in molar equivalent amounts of 0.004 to 0.1, or in molar equivalent amounts of 0.005 to 0.1, or in molar equivalent amounts of 0.01 to 0.05, with respect to the molar amount of formaldehyde.

The base can be an organic base or an inorganic base. Exemplary inorganic bases are alkaline or an alkaline-earth hydroxide, for example, LiOH, NaOH, KOH, or alkaline or an alkaline-earth carbonates and bicarbonates, such as Na₂CO₃, NaHCO₃, K₂CO₃, or KHCO₃, preferably Na₂CO₃ or NaHCO₃. Further bases used can be K₃PO₄ or potassium fluoride. Organic bases can be, for example, amines such as trialkyl amines; for example, trimethyl amine or triethyl amine; or pyridines; as well as acetate salts, such as sodium acetate. Inorganic bases are preferred because the workup is easier (the salts dissolve in the water phase) and have been found to typically provide faster conversions. The base is added in small molar amounts relative to the formaldehyde. For example, it can be added in molar equivalent amounts of 0.001 to 1.0, with respect to the molar amount of formaldehyde, although it is typically added in molar amounts below those of formaldehyde, for example, in molar equivalent amounts of 0.002 to 0.7, or in molar equivalent amounts of 0.003 to 0.5, or in molar equivalent amounts of 0.008 to 0.2, or in molar equivalent amounts of 0.01 to 0.1, or in molar equivalent amounts of 0.02 to 0.08, with respect to the molar amount of formaldehyde. Therefore, contrary to the teachings of US 2020/0010395, the present process preferably uses small catalytic amounts of base.

The temperature of the reaction is not critical, for example, between 0°C and 120°C. The faster the reaction, the less impurities are likely to be formed, but increasing the temperature has the opposite effect, increasing especially the dehydration impurities. It is advisable to use a temperature that strikes a balance between speed and selectivity, and it is typically increased to a temperature between 20°C and 100°C, for example, between 30°C and 80°C, for example between 35°C and 70°C, or between 40°C and 60°C or between 40°C and 50°C.

Typically, the reaction mixture is allowed to react at the chosen temperature for 1 to 48 hours. Typical reaction times are comprised between 1 and 10 hours, for example, less than 8 hours or less than 6 hours, or less than 4 hours, for example, between 1 and 4 hours.

The reaction can proceed in the presence of a solvent, but it is not required and can proceed neat, using the compound of formula (II) as solvent. Thus, the compound of formula (II) is typically added in excess with respect to formaldehyde, for example, such that the molar proportion between formaldehyde and the compound of formula (II) is between 1:10 and 1:1, for example, between 1:9 and 1:2 or between 1:8 and 1:3 or between 1:6 and 1:4. The compounds of formula (II), such as 3-pentanone, are readily available from commercial sources.

Choosing the right proportion between water and the formaldehyde can further improve the yield and the conversion. The inventors have found that an optimal weight proportion between formaldehyde and water is from 5:1 to 1:5, preferably from 4:1 to 1:4, for example from 2:1 to 1:2.

For example, R¹ and R² are each independently selected from the group consisting of hydrogen, C₁-C₁₂ alkyl, C₂-C₁₂ alkenyl, and C₂-C₁₂ alkynyl, for example, C₁-C₆ alkyl groups, for example C₁-C₃ alkyl groups, more preferably ethyl or methyl. It is preferred that R¹ and R² are both methyl, that is, to provide the compound of formula (I) 1-hydroxy-2-methylpentan-3-one. After most of the formaldehyde is converted into compound of formula (I) known procedures are used to separate the product. In a typical reaction set up the compound of formula (II) has been added in excess and is typically removed, for example, through distillation. The compound of formula (II) so separated can be recycled. This process can remove the solvent, if any. Then, the pH is reduced by addition of an acid (e.g. hydrochloric acid or sulfuric acid). The pH is typically reduced to a value between 5 and 10, for example, between 6 and 8 or between 6.5 and 7.5. At this pH the compound of formula (I) can be extracted using common organic solvents.

Formaldehydes of various qualities and forms may be used. For instance, it is possible to feed pure formaldehyde (solid) to the reaction mixture. Formaldehyde sources in aqueous solutions can be used since they are easy to handle. These may be of technical-grade quality and laboratory quality. The formaldehyde content of aqueous solutions is typically between 30 and 60 wt.%. For instance, in the method according to the invention, the formaldehyde can be used in the form of its aqueous solutions having a formaldehyde content of 30 to 60 wt.%. Alternatively, pure paraformaldehyde can be used. Contrary to the teachings of US 2020/0010395, the inventors of the present invention have found paraformaldehyde suitable. Aqueous solutions of formaldehyde also typically comprise methanol as stabilizer. Depending on the quality of the formaldehyde solution, the methanol content can vary in a wide range. Thus, in the present process, aqueous formaldehyde solutions can be used having a methanol content of 0.1 to 20 wt.%, for example of 0.2 to 5 wt.%.

The order of addition of the reagents and starting materials is not critical and typically all are added into the reaction to create a reaction mixture, which is then heated (if needed) and stirred until the reaction is finished. The consumption of the formaldehyde and the evolution of the reaction may be monitored by determining and/or measuring the presence of the starting materials and/or the product formed by means known in the art, e.g., thin layer chromatography (TLC) or high performance liquid chromatography (HPLC).

The resulting compound of formula (I) can be then purified following standard conditions (distillation, gel chromatography, recrystallization, etc...), or can be used directly for the next step.

The present disclosure has described how different variables of the process can be tuned within certain ranges. The present disclosure also encompasses combinations of different ranges of these variables. For example, the skilled person can recognize that the present disclosure covers a process for preparing a compound of formula (I), as defined elsewhere in the present specification, the process comprising the preparation of a compound of formula (I) or a salt thereof, 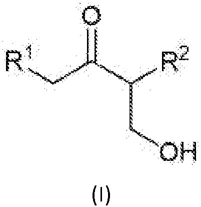
wherein each of R¹ and R² is selected from the group consisting of hydrogen, C₁-C₁₂
alkyl, C₂-C₁₂ alkenyl, C₂-C₁₂ alkynyl, and C₆-C₁₅ aryl;
by contacting a base selected from the group consisting of alkaline or alkaline-earth carbonates or bicarbonates and trialkyl amines, a phase transfer catalyst, formaldehyde and water with a compound of formula (II), 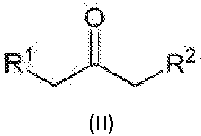 wherein R¹ and R² are as defined above.

For example, the invention refers to a process for the preparation of a compound of formula (I) or a salt thereof, 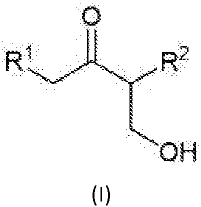
wherein each of R¹ and R² is selected from the group consisting of hydrogen, C₁-C₁₂
alkyl, C₂-C₁₂ alkenyl, C₂-C₁₂ alkynyl, C₆-C₁₅ aryl, C₂-C₁₅ heterocyclyl and C₇-C₁₅ arylalkyl; the process comprising contacting a base, a phase transfer catalyst, paraformaldehyde and water with a compound of formula (II),

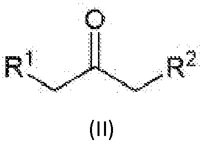

wherein R¹ and R² are as defined above,
wherein the weight proportion between paraformaldehyde and water is between 5:1 and 1:5, preferably between 3:1 and 1:3.

For example, the invention refers to a process for the preparation of a compound of formula (I) or a salt thereof, 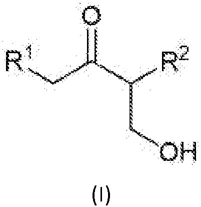
wherein each of R¹ and R² is selected from the group consisting of hydrogen, C₁-C₁₂
alkyl, C₂-C₁₂ alkenyl, and C₂-C₁₂ alkynyl;

the process comprising contacting a base, a phase transfer catalyst, paraformaldehyde and water with a compound of formula (II), 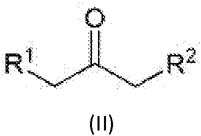
wherein R¹ and R² are as defined above; and
wherein the base is added in molar equivalent amounts of 0.008 to 0.2, with respect to the molar amount of paraformaldehyde.

For example, the invention refers to a process for the preparation of a compound of formula (I) or a salt thereof, 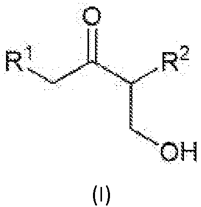
wherein each of R¹ and R² is selected from the group consisting of hydrogen, C₁-C₁₂
alkyl, C₂-C₁₂ alkenyl, C₂-C₁₂ alkynyl, C₆-C₁₅ aryl, C₂-C₁₅ heterocyclyl and C₇-C₁₅ arylalkyl; the process comprising contacting an inorganic base, a phase transfer catalyst, formaldehyde and water with a compound of formula (II),

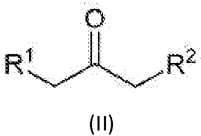

wherein R¹ and R² are as defined above;
wherein the inorganic base is added in molar equivalent amounts of 0.008 to 0.2, with respect to the molar amount of formaldehyde; and
wherein the weight proportion between formaldehyde and water is from 5:1 to 1:5, preferably between 3:1 and 1:3.

For example, the invention refers to a process for the preparation of a compound of formula (I) or a salt thereof, 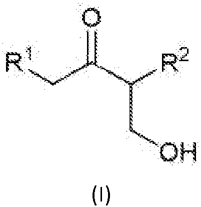 wherein each of R¹ and R² is selected from the group consisting of hydrogen, and a C₁-C₁₂ alkyl, preferably a C₁-C₄-alkyl;
the process comprising contacting an inorganic base selected from the group consisting of alkaline or alkaline-earth carbonates and bicarbonates, for example, sodium carbonate or sodium bicarbonate, a phase transfer catalyst, paraformaldehyde and water with a compound of formula (II), 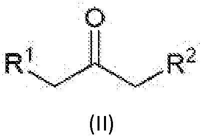
wherein R¹ and R² are as defined above.

For example, the invention refers to a process for the preparation of a compound of formula (I) or a salt thereof, 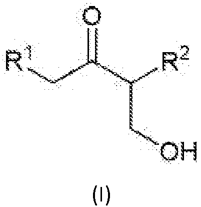 wherein each of R¹ and R² is selected from the group consisting of a C₁-C₄ alkyl group; the process comprising contacting a base selected from the group consisting of alkaline or alkaline-earth carbonates or bicarbonates and trialkyl amines, a phase transfer catalyst, paraformaldehyde and water with a compound of formula (II), 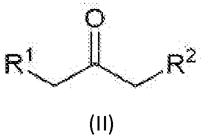
wherein R¹ and R² are as defined above;
wherein the base is added in molar equivalent amounts of 0.008 to 0.2, with respect to the molar amount of formaldehyde; and
wherein the weight proportion between paraformaldehyde and water is from 5:1 to 1:5, preferably between 3:1 and 1:3.

For example, the invention refers to a process for the preparation of 1-hydroxy-2-methylpentan-3-one, the process comprising contacting a base selected from the group consisting of alkaline carbonates or bicarbonates and trialkyl amines, a phase transfer catalyst, paraformaldehyde and water with 3-pentanone, wherein the base is added in molar equivalent amounts of 0.008 to 0.2, with respect to the molar amount of paraformaldehyde.

### EXAMPLES

### Example 1: Preparation of 1-hydroxy-2-methylpentan-3-one (compound of formula (I)) in the presence of water and a phase transfer catalyst according to the invention

3-pentanone (1000.0 g, 6.0 eq.; compound of formula (II)), paraformaldehyde (HCHO)x (58.1 g, 1.0 eq.), Na₂CO₃ (6.2 g, 0.03 eq.), BnTEAC (6.2 g, 0.014 eq.; a phase transfer catalyst) and water (58.1 g) are charged into a 3000 ml three-necked flask. The mixture is stirred at a temperature of 40-45°C for 3 hours, after which the IPC GC-Area% of 1-hydroxy-2-methylpentan-3-one (compound of formula (I)) is 11-13%. The reaction mixture is then cooled down to room temperature and then quenched by adding HCl aq. (6.8 g, conc. HCl 1.2g and H₂O 5.6 g) into the organic layer. The mixture is stirred at room temperature for 20 min., keeping the pH between 6.5 and 7.5. The organic layer is evaporated (50-80°C inner temperature, 200 to 15 mbar) to remove unreacted 3-pentanone and trace water, providing the crude 1-hydroxy-2-methylpentan-3-one as a pale-yellow liquid in 85% yield with respect to the consumed pentanone.

### Example 2: Comparative examples

The same procedure as in Example 1 is repeated for several batches but changing the conditions as indicated in Table 1.

**Table 1**

| Example | Description | Time | Selectivity | Yield based on HCHO | Yield with respect to pentanone consumed |
|---|---|---|---|---|---|
| Comp. 1 | no water and no PTC | 12h | -- | No reaction | No reaction |
| 1 | Example 1 | 3h | 85% | 75% | 85% |
| Comp. 2 | Example 1 with no PTC | 12h | -- | 5% | 5% |
| 2 | With NaHCO₃ as base instead of Na₂CO₃ | 3h | 84% | 65% | 88% |
| 3 | Reaction temp. 80-85°C | 2h | 65% | 80% | 70% |
| 4 | pentanone:FA 3:1 | 3h | 70% | 60% | 72% |
| 5 | pentanone:FA 8:1 | 6h | 85% | 80% | 84% |
| 6 | Dropwise adding aq.FA | 8h | 85% | 75% | 85% |
| 7 | With triethyl amine as base | 3h | 80% | 75% | 80% |

As shown in Table 1, the reaction under the conditions similar to those disclosed in US 5,703,248 shows no conversion at all. Adding water already starts to provide some of the desired 1-hydroxy-2-methylpentan-3-one, although in very low yields (Comp. 2). Good yields are obtained in the presence of a PTC over a range of conditions (entries 1-6). Contrary to the teachings of US 2020/0010395, biphasic reaction conditions provide good yields. For example, the reaction operates well at different proportions between pentanone and paraformaldehyde in water (entries 4 and 5), or with other inorganic bases, such as NaHCO₃ (entry 2) or with organic bases (entry 7). Also, the examples show that the order of addition has little influence in the final outcome (see entry 1 versus entry 6).

### Example 3: Other Phase Transfer Catalysts

Conditions similar to those described in Example 1 were used except for the PTC used. 3-pentanone (20 g, 6.0 eq.; compound of formula (II)), paraformaldehyde (HCHO)x (2.9 g, 1.0 eq.), Na₂CO₃ (0.31 g, 0.03 eq.), phase transfer catalyst (0.01 eq.) and water (2.9 g) are charged into a flask. The mixture is stirred at a temperature of 40-45°C for up to 7.5 hours. During this time the consumption of 3-pentannone and the selectivity towards 1-hydroxy-2-methylpentan-3-one (compound of formula (I)) was measured IPC GC-Area%. The results are shown in Figure 1 and Figure 2.

Figure 1 plots the consumption of the starting 3-pentanone versus time. Since 6 equivalents of 3-pentanone are added with respect to paraformaldehyde, the reaction is theoretically completed once 16.7% (100/6) of 3-pentanone is consumed. Figure 2 plots the selectivity towards the desired compound of formula (I), 1-hydroxy-2-methylpentan-3-one. For all three PTCs tested, TBAB, TBAC and BnTEAC, the conversion was good. Only in the case of TBAC did it change between the second and the seventh hour of reaction. Also, the selectivity was good or excellent. For example, the use of BnTEAC provided an excellent conversion with acceptable selectivity with two hours. Further reaction times did not change the result significantly. On the other hand, TBAB provided acceptable conversion and excellent selectivity. TBAC also provided excellent/acceptable conversion and selectivity, depending on the time chosen to stop the reaction.

## Claims

1. Process for the preparation of a compound of formula (I) or a salt thereof, 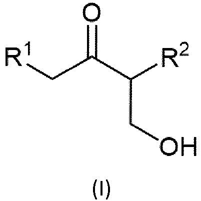
wherein each of R¹ and R² is selected from the group consisting of hydrogen, C₁-C₁₂ alkyl, C₂-C₁₂ alkenyl, C₂-C₁₂ alkynyl, C₆-C₁₅ aryl, C₂-C₁₅ heterocyclyl and C₇-C₁₅ arylalkyl;
the process comprising contacting a base, a phase transfer catalyst, formaldehyde and water with a compound of formula (II) 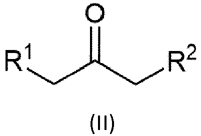
wherein R¹ and R² are as defined above.

2. The process according to claim 1, wherein the reaction proceeds in the presence of a phase transfer catalyst selected from the group consisting of ammonium and phosphonium compounds.

3. The process according to claim 2, wherein the phase transfer catalyst is selected from the group consisting of ammonium compounds.

4. The process according to claim 2, wherein the phase transfer catalyst is selected from the group consisting of tetraalkyl ammonium salts, trialkyl aralkylammonium salts and tetraalkyl-phosphonium salts.

5. The process according to any of claims 3 or 4, wherein the phase transfer catalyst is selected from the group consisting of tetrabutylammonium bromide or chloride, methyl trioctylammonium bromide, methyl tributylammonium chloride, benzyltriethylammonium bromide or chloride, and benzyldodecyldimethylammonium chloride.

6. The process according to any of the previous claims, wherein the phase transfer catalyst is benzyltriethylammonium bromide or benzyltriethylammonium chloride.

7. The process according to any of the previous claims, wherein the phase transfer catalyst is added in molar equivalent amounts of 0.001 to 1.0, with respect to the molar amount of formaldehyde.

8. The process according to any of the previous claims, wherein the base is added in molar equivalent amounts of 0.001 to 1.0, with respect to the molar amounts of formaldehyde.

9. The process according to any of the previous claims, wherein the base is selected from the group consisting of sodium carbonate, sodium bicarbonate and trialkyl amines.

10. The process according to any of the previous claims, wherein the molar proportion between formaldehyde and the compound of formula (II) is between 1:10 and 1:1.

11. The process according to any of the previous claims, wherein the weight proportion between formaldehyde and water is between 5:1 and 1:5.

12. The process according to any of the previous claims, wherein the compound of formula (II) acts as solvent.

13. The process according to any of the previous claims, wherein each of R¹ and R² is selected from the group consisting of C₁-C₆ alkyl groups.

14. The process according to any of the previous claims for the preparation of 1-hydroxy-2-methylpentan-3-one comprising contacting a base, a phase transfer catalyst, formaldehyde and water with 3-pentanone.

15. A process for preparing dimethenamid comprising (i) contacting a base, a phase transfer catalyst, formaldehyde and water with 3-pentanone; and (ii) transforming the 1-hydroxy-2-methylpentan-3-one obtained in the previous step into dimethenamid.

## Patentansprüche

1. Verfahren zur Herstellung einer Verbindung nach Formel (I) oder eines Salzes davon, 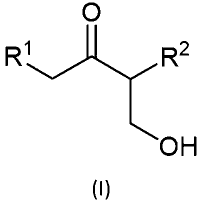
wobei jedes von R¹ und R² ausgewählt ist aus der Gruppe bestehend aus Wasserstoff, C₁-C₁₂ Alkyl, C₂-C₁₂ Alkenyl, C₂-C₁₂ Alkynyl, C₆-C₁₅ Aryl, C₂-C₁₅ Heterocyclyl und C₇-C₁₅ Arylalkyl;
das Verfahren umfassend das Kontaktieren einer Base, eines Phasentransferkatalysators, Formaldehyds und Wassers mit einer Verbindung der Formel (II) 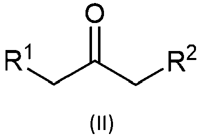
wobei R¹ und R² wie oben definiert sind.

2. Das Verfahren gemäß Anspruch 1, wobei die Reaktion in Anwesenheit eines Phasentransferkatalysators abläuft, der ausgewählt ist aus der Gruppe bestehend aus Ammonium- und Phosphoniumverbindungen.

3. Das Verfahren gemäß Anspruch 2, wobei der Phasentransferkatalysator ausgewählt ist aus der Gruppe bestehend aus Ammoniumverbindungen.

4. Das Verfahren gemäß Anspruch 2, wobei der Phasentransferkatalysator ausgewählt ist aus der Gruppe bestehend aus Tetraalkylammoniumsalzen, Trialkylaralkylammoniumsalzen und Tetraalkyl-Phosphoniumsalzen.

5. Das Verfahren gemäß einem der Ansprüche 3 oder 4, wobei der Phasentransferkatalysator ausgewählt ist aus der Gruppe bestehend aus Tetrabutylammoniumbromid oder Chlorid, Methyltrioctylammoniumbromid, Methyltributylammoniumchlorid, Benzyltriethylammoniumbromid oder Chlorid, und Benzyldodecyldimethylammoniumchlorid.

6. Das Verfahren gemäß einem der vorherigen Ansprüche, wobei der Phasentransferkatalysator Benzyltriethylammoniumbromid oder Benzyltriethylammoniumchlorid ist.

7. Das Verfahren gemäß einem der vorherigen Ansprüche, wobei der Phasentransferkatalysator in molaren äquivalenten Mengen von 0,001 bis 1,0 in Bezug auf die molare Formaldehydmenge hinzugefügt wird.

8. Das Verfahren gemäß einem der vorherigen Ansprüche, wobei die Base in molaren äquivalenten Mengen von 0,001 bis 1,0 bezüglich der molaren Formaldehydmengen hinzugefügt wird.

9. Das Verfahren gemäß einem der vorherigen Ansprüche, wobei die Base ausgewählt ist aus der Gruppe bestehend aus Natriumcarbonat, Natriumbicarbonat und Trialkylaminen.

10. Das Verfahren gemäß einem der vorherigen Ansprüche, wobei das molare Verhältnis zwischen Formaldehyd und der Verbindung der Formel (II) zwischen 1:10 und 1:1 liegt.

11. Das Verfahren gemäß einem der vorherigen Ansprüche, wobei das Gewichtsverhältnis zwischen Formaldehyd und Wasser zwischen 5:1 und 1:5 liegt.

12. Das Verfahren gemäß einem der vorherigen Ansprüche, wobei die Verbindung der Formel (II) als Lösungsmittel wirkt.

13. Das Verfahren gemäß einem der vorherigen Ansprüche, wobei jedes von R¹ und R² ausgewählt ist aus der Gruppe bestehend aus C₁-C₆-Alkylgruppen.

14. Das Verfahren gemäß einem der vorherigen Ansprüche zur Herstellung von 1-Hydroxy-2-Methylpentan-3-on, umfassend das Kontaktieren einer Base, eines Phasentransferkatalysators, Formaldehyds und Wassers mit 3-Pentanon.

15. Ein Verfahren zur Herstellung von Dimethenamid, umfassend (i) Kontaktieren einer Base, eines Phasentransferkatalysators, Formaldehyds und Wassers mit 3-Pentanon; und (ii) Umwandeln des im vorherigen Schritt gewonnenen 1-Hydroxy-2-Methylpentan-3-on in Dimethenamid.

## Revendications

1. Procédé pour la préparation d'un composé de formule (I) ou d'un sel de celui-ci, 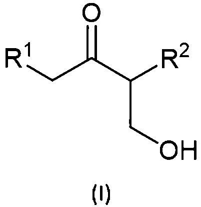
dans lequel chacun des R¹ et R² est choisi parmi le groupe constitué d'hydrogène, d'alkyle en C₁-C₁₂, d'alcényle en C₂-C₁₂, d'alcynyle en C₂-C₁₂, d'aryle en C₆-C₁₅, d'hétérocyclyle en C₂-C₁₅ et d'arylalkyle en C₇-C₁₅ ;
le procédé comprenant la mise en contact d'une base, d'un catalyseur de transfert de phase, de formaldéhyde et d'eau avec un composé de formule (II) 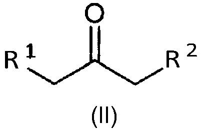
où R¹ et R² sont tels que définis ci-dessus.

2. Le procédé selon la revendication 1, dans lequel la réaction se déroule en présence d'un catalyseur de transfert de phase choisi dans le groupe constitué par les composés d'ammonium et de phosphonium.

3. Le procédé selon la revendication 2, dans lequel le catalyseur de transfert de phase est choisi dans le groupe constitué par les composés d'ammonium.

4. Le procédé selon la revendication 2, dans lequel le catalyseur de transfert de phase est choisi dans le groupe constitué par les sels de tétraalkylammonium, les sels de trialkyl aralkylammonium et les sels de tétraalkylphosphonium.

5. Le procédé selon l'une quelconque des revendications 3 ou 4, dans lequel le catalyseur de transfert de phase est choisi dans le groupe constitué par le bromure ou le chlorure de tétrabutylammonium, le bromure de méthyltrioctylammonium, le chlorure de méthyltributylammonium, le bromure ou le chlorure de benzyltriéthylammonium, et le chlorure de benzyldodécyldiméthylammonium.

6. Le procédé selon l'une quelconque des revendications précédentes, dans lequel le catalyseur de transfert de phase est du bromure de benzyltriéthylammonium ou du chlorure de benzyltriéthylammonium.

7. Le procédé selon l'une quelconque des revendications précédentes, dans lequel le catalyseur de transfert de phase est ajouté en quantités molaires équivalentes de 0,001 à 1,0, par rapport à la quantité molaire de formaldéhyde.

8. Le procédé selon l'une quelconque des revendications précédentes, dans lequel la base est ajoutée en quantités molaires équivalentes de 0,001 à 1,0, par rapport aux quantités molaires de formaldéhyde.

9. Le procédé selon l'une quelconque des revendications précédentes, dans lequel la base est choisie dans le groupe constitué par le carbonate de sodium, le bicarbonate de sodium et les trialkylamines.

10. Le procédé selon l'une quelconque des revendications précédentes, dans lequel le rapport molaire entre le formaldéhyde et le composé de formule (II) est compris entre 1:10 et 1:1.

11. Le procédé selon l'une quelconque des revendications précédentes, dans lequel le rapport pondéral entre le formaldéhyde et l'eau est compris entre 5:1 et 1:5.

12. Le procédé selon l'une quelconque des revendications précédentes, dans lequel le composé de formule (II) agit comme solvant.

13. Le procédé selon l'une quelconque des revendications précédentes, dans lequel chacun des R¹ et R² est choisi dans le groupe constitué par les groupes alkyle en C₁-C⁶.

14. Le procédé selon l'une quelconque des revendications précédentes pour la préparation de 1-hydroxy-2-méthylpentan-3-one, comprenant la mise en contact d'une base, d'un catalyseur de transfert de phase, de formaldéhyde et d'eau avec de la 3-pentanone.

15. Un procédé pour la préparation de diméthénamide comprenant (i) la mise en contact d'une base, d'un catalyseur de transfert de phase, de formaldéhyde et d'eau avec de la 3-pentanone ; et (ii) la transformation de la 1-hydroxy-2-méthylpentan-3-one obtenue à l'étape précédente en diméthénamide.
